(19) Europäisches Patentamt

European Patent Office

Office européen des brevets

(11) **EP 1 180 358 B1**

(12) **EUROPÄISCHE PATENTSCHRIFT**

(45) Veröffentlichungstag und Bekanntmachung des Hinweises auf die Patenterteilung:
**04.05.2005 Patentblatt 2005/18**

(51) Int Cl.$^7$: **A61K 7/32**

(21) Anmeldenummer: **01119299.4**

(22) Anmeldetag: **10.08.2001**

(54) **Verwendung von alkoxylierten ungesättigten Fettsäuren als Antitranspirantien**

Use of alkoxylated unsaturated fatty acids as antiperspirants

Utilisation des acides gras insaturés et alcoxylés comme antitranspirants

(84) Benannte Vertragsstaaten:
**AT BE CH CY DE DK ES FI FR GB GR IE IT LI LU MC NL PT SE TR**

(30) Priorität: **19.08.2000 DE 10040665**

(43) Veröffentlichungstag der Anmeldung:
**20.02.2002 Patentblatt 2002/08**

(73) Patentinhaber:
• **Beiersdorf Aktiengesellschaft**
  **20245 Hamburg (DE)**
• **Bode Chemie GmbH & Co.**
  **22525 Hamburg (DE)**

(72) Erfinder:
• **Wolf, Florian, Dr.**
  **37671 Höxster (DE)**
• **Knieler, Roland, Dr.**
  **21218 Harmstorf (DE)**
• **Keskin, Maike, Dr.**
  **22765 Hamburg (DE)**
• **Pietsch, Hanns, Dr.**
  **20148 Hamburg (DE)**
• **Westphal, Ursula**
  **22299 Hamburg (DE)**

(56) Entgegenhaltungen:
**EP-A- 0 244 144**          **EP-A- 0 663 387**
**WO-A-97/30689**          **DE-A- 19 841 796**
**US-A- 5 976 460**

**Beschreibung**

Die vorliegende Erfindung betrifft die Verwendung an sich bekannter Substanzen als antitranspirierend wirksame Agentien. Insbesondere betrifft die Erfindung die Verwendung dieser Stoffe in kosmetischen oder dermatologischen Zubereitungen.

Körpergerüche in ihrem ursprünglichen Sinn dienen der sozialen Kommunikation. Beispielsweise haben spezielle, von den Duftdrüsen abgesonderte Stoffe (Pheromone) in der Tierwelt einen wesentlichen Einfluß auf Sexual-, Territorial- und Aggressionsverhalten oder sind Ausdruck von Individualität bzw. Rangordnung. Für den Menschen hat diese Art von Verständigung jedoch sehr stark an Bedeutung verloren, wie die vergleichsweise kümmerliche Leistungsfähigkeit des menschlichen Geruchssinns andeutet. Körpergeruch wird in modernen, westlich geprägten Industriegesellschaften als aufdringlich, abstoßend und ungepflegt angesehen. Die Anwendung von kosmetischen oder dermatologischen Desodorantien bietet eine Möglichkeit, unangenehmem Körpergeruch entgegenzuwirken.

Körpergeruch entsteht, wenn der an sich geruchlose frische Schweiß durch Mikroorganismen zersetzt wird. Die grampositiven Bakterien bauen das Sebum, Eiweißstoffe und die gelösten Bestandteile des Schweißes ab. Dabei werden geruchsintensive kurzkettige Fettsäuren und Amine frei. Insbesondere der apokrine Schweiß transportiert auf seinem Weg über die Talgdrüsen und Haarfollikel eine Reihe von Mikroorganismen an die Hautoberfläche, die recht bald in die Zersetzung eingreifen. Auf der Haut befindet sich aber auch eine bestimmte residente Mikroorganismenpopulation, welche, ergänzt durch transiente Mikroorganismen aus der Umwelt, den Abbau des Schweißes beeinflußt.

Den üblichen kosmetischen Desodorantien liegen unterschiedliche Wirkprinzipien zugrunde.

Übliche in Desodorantien eingesetzte Wirkstoffe besitzen beispielsweise antimikrobielle Eigenschaften, die die mikrobielle Zersetzung des frischen Schweißes verhindern. Eine desodorierende Wirkung läßt sich aber auch durch enzyminhibierende, aber nicht antimikrobiell wirkende Substanzen und/oder durch lonenaustauscher erreichen. Kationenaustauscher z. B. sollen Ammoniak, Indol und ähnliche Gerüche absorbieren. Wirkstoffe mit antitranspiranten Eigenschaften hingegen sind solche, die das Schwitzen des menschlichen Körpers reduzieren oder ganz unterdrücken, wobei die Wirkung bei topischer Applikation in der Regel lokal ist.

Die in sogenannten Antitranspirantien eingesetzten Wirkstoffe hemmen in erster Linie die Schweißsekretion, haben aber im allgemeinen auch eine gewisse antibakterielle Wirkung. Sie wirken blockierend auf den Teil des Ausführungsganges der Schweißdrüsen, der durch das Stratum comeum führt. Sie verengen bzw. verschließen die Schweißdrüsen, so daß das Schwitzen ganz oder teilweise unterdrückt wird.

Als antitranspirante Wirkstoffe sind Aluminiumsalze seit langem bekannt. Aluminiumchlorid, Aluminiumchlorhydrat, -nitrat, -sulfat, -acetat usw. werden seit einigen Jahren als antitranspirante oder antiperspirante Wirkstoffe verwendet (siehe WO-A-97/30689). Daneben finden auch Zink-, Magnesium- und Zirkoniumverbindungen Anwendung. Der Stand der Technik wird beispielsweise beschrieben in: *H. P. Fiedler, Der Schweiß, Editio Cantor, Aulendorf, 2. Auflage, S. 303-377, Kapitel K: "Mittel zur Hemmung der Transpiration".* Von den dort als Antitranspirant-Wirkstoffe beschriebenen Metallverbindungen haben sich für die Anwendung in kosmetischen und dermatologischen Antitranspirantien nur die Aluminiumsalze und - in etwas geringerem Maße - die Aluminium/Zirkoniumsalze durchgesetzt.

Der Hauptnachteil der sehr wirksamen Aluminiumchloridlösungen ist, daß sie stark sauer reagieren und daher mitunter ein Brennen auf der Haut und eine Rötung verursachen können, welche eventuell mit Abschilferung verbunden sein kann. Aus diesem Grund setzt man üblicherweise die teilneutralisierten, besser hautverträglichen, aber nicht ganz so wirksamen Aluminiumhydroxychloride ein, die auch einen etwas günstigeren pH-Wert von etwa 4 aufweisen. Allerdings können auch diese bei häufiger Anwendung und bei empfindlichen Personen Hautschäden hervorrufen oder zu unerwünschten Verfärbungen auf Textilien führen. Letzteres kann beispielsweise durch die Verwendung von Zirkonium/Aluminium-Mischsalzen verringert werden.

Antitranspirantien, welche Aluminiumhydroxychloride als Wirkstoffe enthalten, werden vor allem in Form von wäßrigen Lösungen und als Pulver oder Granulate (Trockensprays) eingesetzt. Auch andere Zubereitungsformen, wie z. B. alkoholische Lösungen, Stiftformulierungen, Emulsionen, Mikroemulsionen, Cremes, Salben, Roller und dergleichen, sind prinzipiell denkbar, allerdings teilweise schwierig stabil zu formulieren.

Für Deostifte auf Basis von Natriumstearat oder anderen Seifen beispielsweise sind die sauren Aluminiumsalze nicht geeignet, da sie zum Aussalzen der Seife und somit zu chemischer Unverträglichkeit führen. Bei größeren Zusatzmengen, die für Antitranspirant-Stifte notwendig sind, wird außerdem die Bildung eines festen Seifengels verhindert.

Sollen kosmetische oder pharmazeutische Zubereitungen bestimmte Wirkstoffe enthalten, ist es prinzipiell immer denkbar, daß die übrigen Bestandteile mit den Wirkstoffen nicht kompatibel sind. Aus diesen und aus Gründen der Verträglichkeit ist es daher stets zu bevorzugen, die Einsatzkonzentrationen der Wirkstoffe möglichst niedrig zu halten, selbst bei Verwendung an sich unbedenklicher Substanzen.

Aufgabe der Erfindung war es daher, den Nachteilen des Standes der Technik abzuhelfen und antitranspirante Wirkstoffe zu finden, welche eine gute Wirkung zeigen, nicht so stark sauer sind, eine gute Hautverträglichkeit aufweisen und Textilien nicht schädigen oder verfärben.

Eine weitere Aufgabe der vorliegenden Erfindung war, Zubereitungen zu entwickeln, welche als Grundlage für kosmetische oder dermatologische Desodorantien bzw. Antitranspirantien geeignet sind und sich durch gute Hautverträglichkeit auszeichnen.

Es war indes überraschend und für den Fachmann in keiner Weise vorhersehbar, daß die Verwendung von alkoxylierten ungesättigten Fettsäuren gewählt aus der Gruppe der mindestens einfach ungesättigten, aliphatischen, geradkettigen Fettsäuren mit 8 bis 20 Kohlenstoffatomen, welche einfach bis mehrfach mit 3 bis 20 Ethoxy-, Propoxyund/oder Butoxy-Einheiten alkoxyliert sind,
als antitranspirierend wirksame Agentien die Nachteile des Standes der Technik beseitigt.

Alkoxylierung bzw. Oxalkylierung ist die Bezeichnung für die Insertion einer oder mehrerer Alkoxy-Gruppen, wie beispielsweise Ethoxy-Gruppen (-CH$_2$-CH$_2$-O, auch EO-Gruppen genannt), in Verbindung mit einem aciden Wasserstoff-Atom mit Hilfe von einem Alkylenoxid, wie z. B. Ethylenoxid. Die Produkte der technisch ausgeführten Reaktionen sind lineare Ether bzw. Polyether, die an einem Kettenende eine Hydroxy-Gruppe tragen.

Alkoxylierte ungesättigte Fettsäuren sind handelsübliche Produkte. Sie werden z. B. als fungizide Wirkstoffe (gegen die Pilze Tricophyton, Epidermophyton und Microsporum) zur Herstellung von Mitteln zur Behandlung von Fuß-, Kopf- und Schenkelpilz eingesetzt.

Auch die Verwendung von alkoxylierten ungesättigten Fettsäuren in kosmetischen oder dermatologischen Zubereitungen ist an sich bekannt. Sie werden bevorzugt zur Herstellung von Haarshampoos verwendet.

Bevorzugt im Sinne der vorliegenden Erfindung ist die Verwendung von alkoxylierten ungesättigten Fettsäuren mit 8 bis 14 Kohlenstoffatomen, welche mindestens einfach ethoxyliert sind, insbesondere solchen, welche einfach mit 4 bis 8 Ethoxy-Einheiten alkoxyliert sind.

Erfindungsgemäß besonders bevorzugt ist die Verwendung von mindestens einfach ethoxylierten Undecylensäurealkoxylaten, ganz besonders von solchen, welche mit 3 bis 10 Ethylenoxid-Einheiten alkoxyliert sind, als antitranspirierend wirksame Agentien.

Die Wirkstoffe können erfindungsgemäß vorteilhaft sowohl einzeln als auch im Gemisch vorliegen.

Entsprechend der erfindungsgemäßen Verwendung sind die Zubereitungen besonders vorteilhaft dadurch gekennzeichnet, daß der oder die erfindungsgemäßen antitranspiranten Wirkstoffe in Konzentrationen von 0,01 bis 20,00 Gew.-%, bevorzugt 0,1 bis 10,00 Gew.-%, jeweils bezogen auf das Gesamtgewicht der Zusammensetzung, vorliegt oder vorliegen.

Besonders vorteilhaft sind einfache alkoholische und wäßrige Lösungen, beispielsweise in Ethanol, Propanol-1 sowie Propanol-2, insbesondere solche, welche eine Wirkstoffkonzentration von mehr 0,1 Gew.-%, bezogen auf das Gesamtgewicht der Zusammensetzung, aufweisen. Ebenfalls vorteilhaft ist die erfindungsgemäße Verwendung der Wirkstoffe in Emulsionen und Cremes, welche mindestens eine Fettphase enthalten, wobei die Konzentration der Wirkstoffe in diesen Zubereitungen vorteilhaft aus dem Bereich zwischen 2 und 5 Gew.-%, bezogen auf das Gesamtgewicht der Zubereitungen, gewählt wird.

Stellen die erfindungsgemäß erhaltenen Zubereitungen Desodorantien/Antitranspirantien dar, so können zusätzlich zu den erfindungsgemäß verwendeten alkoxylierten ungesättigten Fettsäuren alle gängigen Wirkstoffe vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäß erhaltenen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den Patentoffenlegungsschriften DE-37 40 186, DE-39 38 140, DE-42 04 321, DE-42 29 707, DE-42 29 737, DE-42 37 081, DE-43 09 372, DE-43 24 219 beschriebenen wirksamen Agenzien.

Auch die Verwendung weiterer üblicher Antitranspiranswirkstoffe kann vorteilhaft im Sinne der vorliegenden Erfindung sein, insbesondere die Verwendung von Adstringentien, beispielsweise von basischen Aluminiumchloriden in erfindungsgemäßen, antitranspirierend wirksamen Zubereitungen.

Entsprechend der erfindungsgemäßen Verwendung können die kosmetischen Zubereitungen als Desodorantien bzw. Antitranspirantien bezeichnet werden. Vorteilhaft können sie beispielsweise in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, als Deo-Stifte (Deo-Sticks) und in Form von aus normalen Flaschen und Behältern auftragbaren W/Ooder O/W-Emulsionen, z.B. Crèmes oder Lotionen. Weiterhin können die kosmetischen Desodorantien vorteilhaft in Form von desodorierenden bzw. antitranspirierend wirkenden Tinkturen, desodorierenden bzw. antitranspirierend wirkenden Intimreinigungsmitteln, desodorierenden bzw. antitranspirierend wirkenden Pudern oder deodorierenden bzw. antitranspirierend wirkenden Pudersprays vorliegen.

Als Treibmittel für aus Aerosolbehältem versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Als übliche kosmetische Trägerstoffe zur Herstellung der deodorierenden bzw. antitranspirierend wirkenden Zubereitungen gemäß der erfindungsgemäßen Verwendung können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z. B. Hydroxyethyloder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosität.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Camosin, L-Carnosin und deren Derivate (z.B. Anserin), Carotinoide, Carotine (z.B. $\alpha$-Carotin, $\beta$-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Alanindiessigsäure, Flavonoide, Polyphenole, Catechine, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoäharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern die kosmetische oder dermatologische Zubereitung im Sinne der vorliegenden Erfindung eine Lösung oder Emulsion oder Dispersion darstellt, können als Lösungsmittel verwendet werden:

- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Emulsionen entsprechend der vorliegenden Erfindungen enthalten einen oder mehrere Emulgatoren, insbesondere vorteilhaft gewählt aus der Gruppe Glycerylstearat im Gemisch mit Ceteareth-20; Sorbitanstearat; Sorbitanoleat; Ceteareth-25; Ceteareth-6 im Gemisch mit Stearylalcohol; Cetylstearylalcohol im Gemisch mit PEG-40-Ricinusöl und Natriumcetylstearylsulfat; Triceteareth-4 Phosphat; Glycerylstearat; Natriumcetylstearylsulfat; Lecithin; Trilaureth-4 Phosphat; Laureth-4 Phosphat; Stearinsäure; Propylenglycolstearat SE; PEG-25-hydriertes Ricinusöl; PEG-54-hydriertes Ricinusöl; PEG-6 Caprylsäure/Caprinsäureglyceride; Sorbitanstearat; Glyceryloleat im Gemisch mit Propylenglycol; PEG-9-Stearat; Glyceryllanolat; Ceteth-2; Ceteth-20; Polysorbat 60; Lanolin; Glycerylstearat im Gemisch mit PEG-100 Stearat; Glycerylmyristat; mikrokristallines Wachs (Cera microcristallina) im Gemisch mit Paraffinöl (Paraffinum liquidum), Ozokerit, hydriertem Ricinusöl, Glyceryl Isostearat und Polyglyceryl-3-Oleat; Glyceryllaurat; PEG-40-Sorbitanperoleat; Laureth-4; Ceteareth-3; Wollwachssäuregemische; Isostearylglycerylether; Cetylstearylalkohol im Gemisch mit Natrium Cetylstearylsulfat; Wollwachsalkoholgemische; Laureth-23; Steareth-2; Glycerylstearat im Gemisch mit PEG-30 Stearat; PEG-40-Stearat; Glycol Distearat; PEG-22-Dodecyl Glycol Copolymer; Polyglyceryl-2-PEG-4 Stearat; Pentaerythrithylisostearat; Polyglyceryl-3 Diisostearat; Ceteareth-20; Sorbitan Oleat im Gemisch mit hydriertem Ricinusöl, Bienenwachs (Cera alba) und Stearinsäure; Natriumdihydroxycetylphosphat im Gemisch mit Isopropylhydroxycetylether; Methylglucosesesquistearat; Steareth-10; PEG-20-Stearat; Steareth-2 im Gemisch mit PEG-8 Distearat; Steareth-21; Steareth-20; Isosteareth-20; Methylglucosedioleat; PEG-7-hydriertes Ricinusöl; Sorbitanoleat im Gemisch mit PEG-2-hydriertem Ricinusöl, Ozokerit und hydriertem Ricinusöl; Sorbitanisostearat im Gemisch mit PEG-2-hydriertem Ricinusöl, Ozokerit und hydriertem Ricinusöl; PEG-45/ Dodecylglycol-Copolymer; Methoxy-PEG-22/Dodecylglycol-Copolymer; hydrierte Cocosfettsäureglyceride; Polyglyceryl-4-Isostearat; PEG-40-Sorbitanperoleat; PEG-40-Sorbitanperisostearat; PEG-20-Glycerylstearat; PEG-20-Glycerylstearat; PEG-8-Bienenwachs; Laurylmethiconcopolyol; Cetyldimethiconcopolyol; Polyglyceryl-2-laurat; Isostearyldiglycerylsuccinat; Stearamidopropyl-PG-dimoniumchloridphosphat; PEG-7-hydriertes Ricinusöl; Glycerylstearat, Ceteth-20; Triethylcitrat; PEG-20-Methylglucosesesquistearat; Ceteareth-12; Paraffinöl (Paraffinum liquidum); Glycerylstearatcitrat; Cetylphosphat; Sorbitansesquioleat; Acrylat/$C_{10-30}$-Alkylacrylat-Crosspolymer; Sorbitanisostearat; Methylglucosesesqui-

stearat; Triceteareth-4-Phosphat; Trilaureth-4-Phosphat; Polyglycerylmethylglucosedistearat; Poloxamer 101; Kaliumcetylphosphat; Isosteareth-10; Polyglyceryl-2-sesquiisostearat; Ceteth-10; Polyglyceryl-2 Dipolyhydroxystearat; Oleth-20; Isoceteth-20; Glycerylisostearat; Polyglyceryl-3-Diisostearat; Glycerylstearat im Gemisch mit Ceteareth-20, Ceteareth-12, Cetylstearylalcohol und Cetylpalmitat; Cetylstearylalcohol im Gemisch mit PEG-20 Stearat; Glycerylstearat; PEG-30-Stearat.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Camaubawachs, Candelillawachs)

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Zubereitungen gemäß der vorliegenden Erfindung können sich auch durch einen Gehalt an Tensiden auszeichnen.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielsweise $-COO^-$, $-OSO_3^{2-}$, $-SO_3^-$, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:

- anionische Tenside,
- kationische Tenside,
- amphotere Tenside und
- nichtionische Tenside.

Anionische Tenside weisen als funktionelle Gruppen in der Regel Carboxylat-, Sulfatoder Sulfonatgruppen auf. In wäßriger Lösung bilden sie im sauren oder neutralen Milieu negativ geladene organische Ionen. Kationische Tenside sind beinahe ausschließlich durch das Vorhandensein einer quartemären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| $RNH_2^+CH_2CH_2COOH\ X^-$ | (bei pH=2) | X' = beliebiges Anion, z.B. $Cl^-$ |
| $RNH_2^+CH_2CH_2COO^-$ | (bei pH=7) | |
| $RNHCH_2CH_2COO^-\ B^+$ | (bei pH=12) | $B^+$ = beliebiges Kation, z.B. $Na^+$ |

Typisch für nicht-ionische Tenside sind Polyether-Ketten. Nicht-ionische Tenside bilden in wäßrigem Medium keine Ionen.

A. Anionische Tenside

Vorteilhaft zu verwendende anionische Tenside sind

Acylaminosäuren (und deren Salze), wie

1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Alaninate

Carbonsäuren und Derivate, wie

1. Carbonsäuren, beispielsweise Laurinsäure, Aluminiumstearat, Magnesiumalkanolat und Zinkundecylenat, und Derivate, wie z. B. Acyllactylate, Lauroyllactylat, Caproyllactylat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,
**[0057]** Sulfonsäuren und Salze, wie

1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium $C_{12-14}$ Olefinsulfonat, Natriumlauryl-sulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat

sowie
Schwefelsäureester, wie

1. Alkylethersulfat, beispielsweise Natrium-, Ammonium-, Magnesium-, MIPA-, TIPA-Laurethsulfat, Natriummy-rethsulfat und Natrium $C_{12-13}$ Parethsulfat,
2. Alkylsulfate, beispielsweise Natrium-, Ammonium- und TEA- Laurylsulfat.

B. Kationische Tenside

Vorteilhaft zu verwendende kationische Tenside sind

1. Alkylamine,
2. Alkylimidazole,
3. Ethoxylierte Amine und
4. Quaternäre Tenside.
5. Esterquats

Zubereitungen gemäß der vorliegenden Erfindung können ferner vorteilhaft quatemäre Ammoniumverbindungen enthalten, insbesondere solche, welche als quatemäre Tenside bezeichnet werden. Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft zu verwenden sind Alkylbetain, Alkylamidopropylbetain und Alkylamidopropylhydroxysulfain, Alkylamidethyltrimethylammoniumethersulfate, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

C. Amphotere Tenside

Vorteilhaft zu verwendende amphotere Tenside sind

1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

D. Nicht-ionische Tenside

Vorteilhaft zu verwendende nicht-ionische Tenside sind

1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

Der Nachweis der Wirksamkeit wurde an menschlichen Probanden mit Hilfe der sogenannten "Imprintmethode" geführt. Bei dieser Methode wird das zu untersuchende Material am Unterarm innen mehrere Tage hintereinander (topisch) angewendet, sodann begeben sich die Probanden in die Sauna zum Schwitzen. An der Untersuchungsstelle wird eine Silikonmasse aufgetragen, die während der Schwitzphase aushärtet. An den Stellen, an denen Schweißtröpfchen austreten, bilden sich Abdrücke in der Silikonmasse, welche bildanalytisch ausgewertet werden können. Nach diesem Verfahren wurden zunächst die Wirkstoffe in wäßriger Lösung untersucht und sodann entsprechende kosmetische Zubereitungen.

Mit Hilfe der Imprintmethode läßt sich die sogenannte "Reduktion der Schweißdrüsen" bestimmen. Dazu wird der Test einmal vor und einmal nach der Behandlung mit einem Antitranspirant durchgeführt und jeweils die Zahl n der aktiven Schweißdrüsen ausgezählt. Die Reduktion der Schweißdrüsen ergibt sich dann nach der Formel:

$$\frac{[n(vorher) - n(nachher)] \times 100}{n(vorher)}$$

**Beispiele:**

**Beispiele 1-3**

Es wurden wäßrige Lösungen aus Undecylensäurepolyethylenglycolester hergestellt und an 15 Probanden die Schweißreduktion mit Hilfe der vorstehend beschriebenen Imprint Methode am inneren Unterarm geprüft. Zum Vergleich wurde eine 10 %ige wäßrige Aluminiumchlorhydrat-Lösung verwendet.

**Ergebnisse**

**[0068]**

| | |
|---|---|
| 10 % Alchlorhydratlsg. | 83 % Reduktion an aktiven Schweißdrüsen |
| 5 % Undecylensäurepolyethylenglycolester (6 EO) | 32 % Reduktion |
| 10 % Undecylensäurepolyethylenglycolester (6 EO) | 50 % Reduktion |
| 20 % Undecylensäurepolyethylenglycolester (6 EO) | 60 % Reduktion |

**Beispiel 4**

**Antitranspiranter Fußzerstäuber**

**[0069]**

10,00 Gew. Teile Undecylensäurepolyethylenglycolester (6 EO)
60,00 Gew. Teile Ethanol, wasserfrei
0,10 Gew. Teil Menthol
0,25 Gew. Teile Fettalkoholpolyglycolether (5 EO)
0,02 Gew. Teile Allantoin
0,10 Gew. Teil Parfum
29,53 Gew. Teile aqua purificata

**[0070]** Dieses Präparat wurde wie in den Beispielen 1-3 an Hand der Imprint Methode an 15 Probanden am inneren Unterarm auf seine antitranspirante Wirkung getestet. Ergebnis: 7 % Reduktion der aktiven Schweißdrüsen. Außerdem wurde geprüft ob eine Hautreizung erfolgt oder allergische Reaktionen auftreten. Ergebnis: keine.

**Beispiel 5**

**Antitranspiranter Fußzerstäuber**

**[0071]**

20,00 Gew. Teile Undecylensäurepolyethylenglycolester (6 EO)
60,00 Gew. Teile Ethanol, wasserfrei
0,10 Gew. Teil Menthol
0,25 Gew. Teile Fettalkoholpolyglykolether (5 EO)
0,02 Gew. Teile Allantoin
0,10 Gew. Teil Parfum
19,52 Gew. Teile aqua purificata

**[0072]** Dieses Präparat wurde wie in den Beispielen 1-4 an Hand der Imprint Methode an 15 Probanden am inneren Unterarm auf seine antitranspirante Wirkung getestet. Ergebnis: 23 % Reduktion der aktiven Schweißdrüsen. Außerdem wurde geprüft ob eine Hautreizung erfolgt oder allergische Reaktionen auftreten. Ergebnis: keine.

**[0073]** Dieses Präparat wurde wie in den Beispielen 1-5 an Hand der Imprint Methode an 15 Probanden am inneren Unterarm auf seine antitranspirante Wirkung getestet. Ergebnis: 5 % Reduktion der aktiven Schweißdrüsen. Außerdem wurde geprüft ob eine Hautreizung erfolgt oder allergische Reaktionen auftreten. Ergebnis: keine.

**Beispiel 6**

**Antitranspirante Creme**

**[0074]**

10,00 Gew. Teile Undecylensäurepolyethylenglycolester (6 EO)
05,00 Gew. Teile Decamethylcyclopentasiloxan
00,20 Gew. Teile Lanolinalkohol

00,50 Gew. Teile C12-C15 Alkylbenzoat
02,50 Gew. Teile 1-Hexadecanol
03,50 Gew. Teile Polyoxyethylenstearat (40 EO)
05,00 Gew. Teile Paraffinum subliquidum
03,00 Gew. Teile Gemisch von Mono- und Diglyceriden der Stearin- und Palmitinsäure
69,80 Gew. Teile Aqua purificata
00,25 Gew. Teile p-Hyroxybenzoesäure methylester

Dieses Präparat wurde wie in den Beispielen 1-3 an Hand der Imprint Methode an 15 Probanden am inneren Unterarm auf seine antitranspirante Wirkung getestet. Ergebnis: 13 % Reduktion der aktiven Schweißdrüsen. Außerdem wurde geprüft ob eine Hautreizung erfolgt oder allergische Reaktionen auftreten. Ergebnis: keine.

**Beispiel 7**

**Antitranspirante Creme**

20,00 Gew. Teile Undecylensäurepolyethylenglycolester (6 EO)
05,00 Gew. Teile Decamethylcyclopentasiloxan
00,20 Gew. Teile Lanolinalkohol
00,50 Gew. Teile $C_{12}$-$C_{15}$ Alkylbenzoat
02,50 Gew. Teile 1-Hexadecanol
03,50 Gew. Teile Polyoxyethylenstearat (40 EO)
05,00 Gew. Teile Paraffinum subliquidum
03,00 Gew. Teile Gemisch von Mono- und Diglyceriden der Stearin- und Palmitinsäure
59,80 Gew. Teile Aqua purificata
00,25 Gew. Teile p-Hyroxybenzoesäuremethylester

Dieses Präparat wurde wie in den Beispielen 1-3 an Hand der Imprint Methode an 15 Probanden am inneren Unterarm auf seine antitranspirante Wirkung getestet. Ergebnis: 7 % Reduktion der aktiven Schweißdrüsen. Außerdem wurde geprüft ob eine Hautreizung erfolgt oder allergische Reaktionen auftreten. Ergebnis: keine.

**Beispiel 8**

**Antitranspirantes wäßriges Gel**

30,00 Gew.Teile Undecylensäurepolyethylenglycolester (6 EO)
03,00 Gew. Teil Polyacrylamid
01,50 Gew. Teile Isoparaffinöl
00,42 Gew. Teile Dodecylalkoholpolyethoxylat (7 EO)
01,00 Gew. Teile Rosmarinöl
00,25 Gew. Teile p-Hydroxybenzoesäuremethylester
63,83 Gew. Teile Aqua purificata

Dieses Präparat wurde wie in den Beispielen 1-3 an Hand der Imprint Methode an 15 Probanden am inneren Unterarm auf seine antitranspirante Wirkung getestet. Ergebnis: 22 % Reduktion der aktiven Schweißdrüsen. Außerdem wurde geprüft ob eine Hautreizung erfolgt oder allergische Reaktionen auftreten. Ergebnis: keine.

**Beispiel 9**

**Antitranspirante Mikroemulsion**

15,00 Gew. Teile Undecylensäurepoyethylenglycolester (6 EO)

04,50 Gew. Teile Capryl/Caprinsäureester von ungesättigten Fettalkoholen
04,50 Gew. Teile Di-n-Octylether
02,70 Gew.-Teile Glycerinmonostearat
00,36 Gew. Teile Stearylalkoholpolyethylenglycolether (12EO)
00,32 Gew. Teile Fettalkohol $C_{12}$-$C_{18}$
00,22 Gew. Teile Cetylpalmitat
01,90 Gew. Teile Fettalkohol $C_{12}$-$C_{18}$Polyethylengycolether (20 EO)
01,00 Gew. Teil Pentandiol-1,3
00,75 Gew. Teile Dexpanthenol
00,24 Gew. Teile Phenoxyethanol
00,06 Gew. Teile Methyldibromoglutarnitril
00,20 Gew. Teile Allantoin

Dieses Präparat wurde wie in den Beispielen 1-3 an Hand der Imprint Methode an 15 Probanden am inneren Unterarm auf seine antitranspirante Wirkung getestet. Ergebnis: 10 % Reduktion der aktiven Schweißdrüsen. Außerdem wurde geprüft ob eine Hautreizung erfolgt oder allergische Reaktionen auftreten. Ergebnis: keine.

**Beispiel 10**

Es wurde eine antitranpirante Fußcreme hergestellt, bestehend aus

4,00 % Undecylensäurepolyethylenglycolester(6EO)
4,00 % Glycerol
9,50 % Tetradecanol
82,50 % demineralisiertes Wasser

**Beispiel 11**

Es wurde ein antitranspiranter Spray hergestellt, bestehend aus

1,50 % Undecylensäurepolyethylenglycolester(6EO)
1,00 % Dexpanthenol
0,50 % Benzoesäure
0,10 % Parfüm
96,90 % Demineralisiertes Wasser

**Beispiel 12**

Es wurde ein antitranspiranter Roll-on Stift hergestellt, bestehend aus

2,00 % Undecylensäurepolyethylenglycolester(6EO)
1,00 % Triethylcitrat
0,50 % Allantoin
0,10 % Parfüm
0,05 % Citronensäure
96,35 % demineralisiertem Wasser

**Beispiel 13**

Es wurde ein antitranspiranter Spray hergestellt, bestehend aus

5,00 % Undecylensäurepolyethylenglycolester(6EO)
15,00 % Ethanol
1,00 % Dexpanthenol
84,00 % demineralisiertem Wasser

**Patentansprüche**

1. Verwendung von alkoxylierten ungesättigten Fettsäuren gewählt aus der Gruppe der mindestens einfach ungesättigten, aliphatischen, geradkettigen Fettsäuren mit 8 bis 20 Kohlenstoffatomen, welche einfach bis mehrfach mit 3 bis 20 Ethoxy-, Propoxyund/oder Butoxy-Einheiten alkoxyliert sind, als antitranspirierend wirksame Agentien.

2. Verwendung nach Anspruch 1, **dadurch gekennzeichnet, daß** der oder die antitranspirierend wirksamen Agentien gewählt werden aus der Gruppe der alkoxylierten ungesättigten Fettsäuren mit 8 bis 14 Kohlenstoffatomen, welche mindestens einfach ethoxyliert sind.

3. Verwendung nach Anspruch 1, **dadurch gekennzeichnet, daß** der oder die antitranspirierend wirksamen Agentien gewählt werden aus der Gruppe der alkoxylierten ungesättigten Fettsäuren mit 8 bis 14 Kohlenstoffatomen, welche einfach mit 4 bis 8 Ethoxy-Einheiten alkoxyliert sind.

4. Verwendung nach Anspruch 1, **dadurch gekennzeichnet, daß** die alkoxylierte ungesättigte Fettsäure oder die alkoxylierten ungesättigten Fettsäuren in Konzentrationen von 0,01 bis 20,00 Gew.-%, bevorzugt 0,1 bis 10,00 Gew.-% vorliegt oder vorliegen, jeweils bezogen auf das Gesamtgewicht der Zusammensetzung.

5. Verwendung nach Anspruch 1, **dadurch gekennzeichnet, daß** weitere Antitranspiranswirkstoffe zugegen sind.


**Claims**

1. Use of akoxylated unsaturated fatty acids chosen from the group of at least monounsaturated, aliphatic, straight-chain fatty acids having 8 to 20 carbon atoms which are mono- to polyalkoxylated with 3 to 20 ethoxy, propoxy and/or butoxy units as antiperspirant agents.

2. Use according to Claim 1, **characterized in that** the antiperspirant agent(s) is/are chosen from the group of alkoxylated unsaturated fatty acids having 8 to 14 carbon atoms which are at least monoethoxylated.

3. Use according to Claim 1, **characterized in that** the antiperspirant agent(s) is/are chosen from the group of alkoxylated unsaturated fatty acids having 8 to 14 carbon atoms which are monoalkoxylated with 4 to 8 ethoxy units.

4. Use according to Claim 1, **characterized in that** the alkoxylated unsaturated fatty acid or the alkoxylated unsaturated fatty acids is or are present in concentrations of from 0.01 to 20.00% by weight, preferably 0.1 to 10.00% by weight, in each case based on the total weight of the composition.

5. Use according to Claim 1, **characterized in that** further antiperspirant active ingredients are present.


**Revendications**

1. Utilisation d'acides gras insaturés alcoxylés choisis parmi le groupe des acides gras linéaires aliphatiques au moins mono-insaturés, ayant de 8 à 20 atomes de carbone, qui sont mono- à poly-alcoxylés avec de 3 à 20 motifs éthoxy, propoxy et/ou butoxy, en tant qu'agents à action antitranspirante.

2. Utilisation selon la revendication 1, **caractérisée en ce que** le ou les agents à action antitranspirante sont choisis parmi le groupe des acides gras insaturés alcoxylés ayant de 8 à 14 atomes de carbone, qui sont au moins mono-éthoxylés.

3. Utilisation selon la revendication 1, **caractérisé en ce que** le ou les agents à action antitranspirante sont choisis parmi le groupe des acides gras insaturés alcoxylés ayant de 8 à 14 atomes de carbone, qui sont mono-alcoxylés avec de 4 à 8 motifs éthoxy.

4. Utilisation selon la revendication 1, **caractérisée en ce que** l'acide gras insaturé alcoxylé ou les acides gras insaturés alcoxylés est ou sont présent(s) en concentrations de 0,01 à 20,00 % en poids, de préférence de 0,1 à 10,00 % en poids, dans chaque cas par rapport au poids total de la composition.

**5.** Utilisation selon la revendication 1, **caractérisée en ce que** des ingrédients actifs anti-transpirants supplémentaires sont ajoutés.